# EUROPÄISCHE PATENTSCHRIFT

(11) **EP 0 719 757 B1**
(45) Veröffentlichungstag und Bekanntmachung des Hinweises auf die Patenterteilung: **15.09.1999**
(21) Anmeldenummer: 96100019.7
(22) Anmeldetag: 02.01.1996
(51) Int. Cl.: C07C 63/68, C07C 51/573

(54) **Verfahren zur Herstellung von 3-Chlorphthalsäureanhydrid**
Process for preparing 3-chlorophthalic anhydride
Procédé de préparation d'anhydride d'acide 3-chlorophtalique

(30) Priorität: 02.01.1995 DE 19500031
(43) Veröffentlichungstag der Anmeldung: 03.07.1996
(73) Patentinhaber: BASF AKTIENGESELLSCHAFT, 67056 Ludwigshafen (DE)
(72) Erfinder: Baur, Karl G., Dr., D-67063 Ludwigshafen (DE); Brunner, Erwin, Dr., D-69469 Weinheim (DE); Brandt, Eckhardt, Dr., D-67105 Schifferstadt (DE)
(74) Vertreter: Isenbruck, Günter, Dr.

(56) Entgegenhaltungen:
- DE-C- 638 200

## Beschreibung

Die vorliegende Erfindung betrifft ein Verfahren zur Herstellung von im wesentlichen reinem 3-Chlorphthalsäureanhydrid aus einem Gemisch, in dem 3-Chlorphthalsäureanhydrid neben 4,5-Dichlorphthalsäureanhydrid vorliegt.

Monochlorierte Phthalsäureanhydride sind aufgrund ihrer Reaktivität gesuchte Zwischenprodukte beispielsweise zur Herstellung von Heterocyclen, als Farbstoffvorprodukte und neuerdings auch zur Herstellung von Polymeren. Für diese Verwendungszwecke werden sie meist in hoher Reinheit benötigt. Während die Herstellung von 4-Chlorphthalsäureanhydrid auf einfache Weise in hoher Ausbeute durch Chlorierung mit Natriumhypochlorit bekannt ist (GB-B 628 401, vgl. auch E. E. Ayling, J. Chem. Soc., 253 (1929)), ist die Herstellung von reinem 3-Chlorphthalsäureanhydrid schwierig.

Reines 3-Chlorphthalsäureanhydrid kann durch Oxidation von 3-Chlor-o-xylol beispielsweise mit Salpetersäure bei erhöhter Temperatur und erhöhtem Druck und anschließende Umwandlung zum Anhydrid der bei der Oxidation entstandenen 3-Chlorphthalsäure erhalten werden. Das isomerenreine 3-Chlor-o-xylol wird durch destillative Auftrennung der Produkte der Kernchlorierung von o-Xylol erhalten, aber diese destillative Auftrennung ist wegen der geringen Siedepunktsunterschiede (relativen Flüchtigkeiten) des 3- und 4-Chlor-o-xylols äußerst aufwendig. Eine technische Kolonne zur gleichzeitigen Herstellung von 3-Chlor-o-xylol und 4-Chlor-o-xylol in Reinheiten von jeweils über 99% bei kontinuierlichem Betrieb benötigt ungefähr 250 theoretische Trennstufen. Andere Trennverfahren zur Auftrennung der Isomeren, beispielsweise fraktionierte Kristallisation, sind nicht weniger aufwendig.

3-Chlorphthalsäureanhydrid kann auch aus 3-Nitrophthalsäureanhydrid durch Austausch der Nitrogruppe gegen Chlor hergestellt werden. Das hierzu benötigte 3-Nitrophthalsäureanhydrid wird in drei Verfahrensstufen durch Nitrierung von Phthalsäureanhydrid mit mäßiger Ausbeute, durch Isomerentrennung der entstandenen Nitrophthalsäuren, durch fraktionierte Kristallisation und durch Umwandlung der erhaltenen 3-Nitrophthalsäure zum Anhydrid hergestellt (M. S. Newman, P. G. Scheurer, J. Am. Chem. Soc. 78, 5005, 1956; Organic Syntheses, Coll. Vol. 1, 1932, 399 - 401). Dieser vielstufige und aufwendige Weg, der zudem schlechte Ausbeuten liefert, ist für eine industrielle Anwendung wenig geeignet.

Die einfachste Synthese der monochlorierten Phthalsäureanhydride, nämlich die Chlorierung von Phthalsäureanhydrid unter Verwendung von Lewis-Säure-Katalysatoren, führt nur bei sehr geringen Umsätzen zu Gemischen, die lediglich die beiden isomeren, durch Destillation trennbaren 3- und 4-Chlorphthalsäureanhydride enthalten; sobald die Umsatzraten wirtschaftlich annehmbare Werte erreichen, entstehen stets auch höher chlorierte Phthalsäureanhydride (A. Zweig und M. Epstein, J. Org. Chem. 43, No. 19, 1978, Seiten 3690 - 3692). Nach dem bisherigen Wissensstand erschien eine Gewinnung von im wesentlichen reinem 3-Chlorphthalsäureanhydrid aus diesem Gemisch unter wirtschaftlich annehmbaren Bedingungen, d.h. durch destillative Abtrennung oder ein anderes großtechnisch durchführbares Verfahren, nicht möglich.

Die Schwierigkeit bei der destillativen Auftrennung des Produktgemisches einer Chlorierung von Phthalsäureanhydrid mit Lewis-Säure-Katalysatoren liegt in der Trennung von 3-Chlorphthalsäureanhydrid und 4,5-Dichlorphthalsäureanhydrid, die laut Literatur genau die gleichen Siedepunkte, bei Normaldruck 313°C, aufweisen. A. Zweig und M. Epstein, loc. cit., konnten nur Mischungen dieser beiden Chlorierungsprodukte erhalten.

Die folgende Tabelle I gibt einen Überblick über die in der Literatur angegebenen Siedepunkte bei Normaldruck und Festpunkte der bei der Chlorierung von Phthalsäureanhydrid anfallenden Produkte.

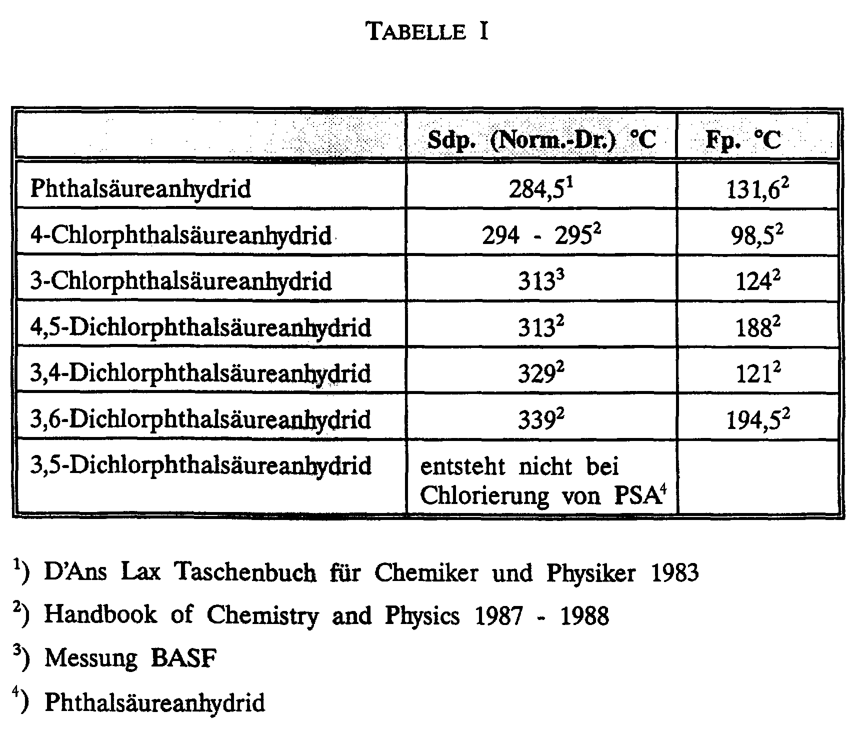

Aus der Tabelle geht hervor, daß die Siedepunkte der von 4,5-Dichlorphthalsäureanhydrid verschiedenen dichlorierten Reaktionsprodukte deutlich höher liegen, so daß deren Abtrennung durch Destillation ohne Schwierigkeiten möglich ist. Auch die Abtrennung von Phthalsäureanhydrid und reinem 4-Chlorphthalsäureanhydrid aus dem Produktgemisch ist Stand der Technik; insbesondere das wirtschaftlich interessante 4-Chlorphthalsäureanhydrid kann durch entsprechende Wahl der Destillationsparameter in hoher Reinheit erhalten werden. Aufgrund der hohen Schmelzpunkte der anfallenden Produkte sind die unter Veränderung des Drucks anwendbaren Siedetemperaturbereiche eingeengt.

A. Zweig und M. Epstein berichten weiter, daß die Auftrennung von 3-Chlorphthalsäureanhydrid und 4,5-Dichlorphthalsäureanhydrid auch durch fraktioniertes Umkristallisieren, fraktioniertes Schmelzen, Extraktion und Sublimation nicht erfolgreich vorgenommen werden kann. Diesen Autoren gelang die Auftrennung lediglich mittels einer polaren Gas-Flüssigkeit-Chromatographiesäule, was für eine Herstellung im industriellen Maßstab unannehmbar ist.

Wie oben erwähnt untersuchten diese Autoren auch das Produktverhältnis der Chlorierung in Abhängigkeit der Reaktionszeit, d. h. des Umsatzes an Phthalsäureanhydrid. Die folgende Tabelle II, die aus A. Zweig und M. Epstein, loc. cit., entnommen ist, verdeutlicht, daß bei höheren Umsätzen von Phthalsäureanhydrid bei einer Chlorierung mit Chlor und Eisen(III)chlorid in 1,1,2,2-Tetrachlorethan der Anteil der monochlorierten Produkte zugunsten des Anteils der höher chlorierten Produkte abnimmt, was auf die etwa gleiche Reaktivität der monochlorierten Produkte und von Phthalsäureanhydrid selbst zurückgeführt wird.

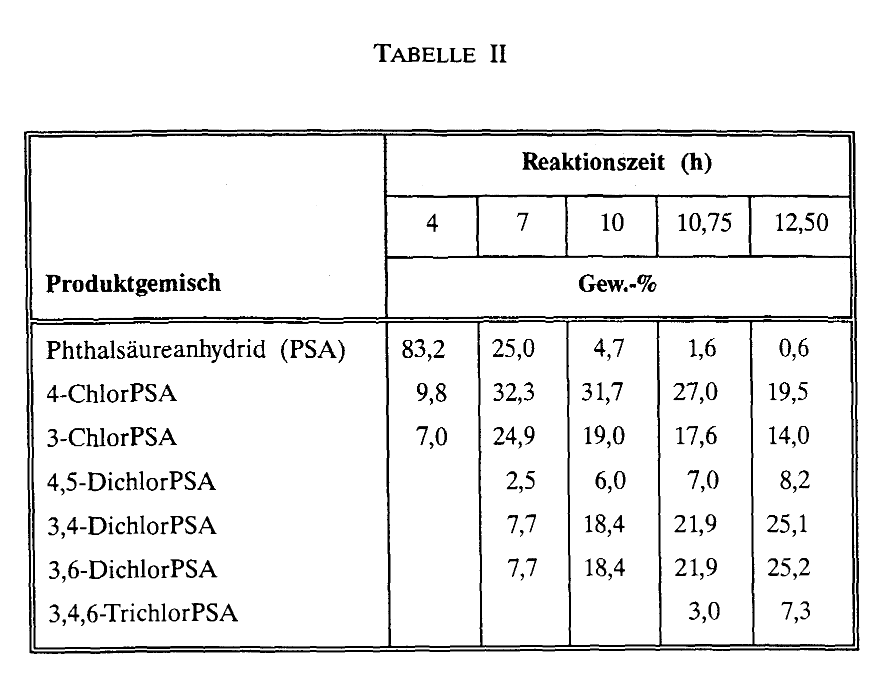

Die obigen Befunde ließen es bisher unmöglich erscheinen, 3-Chlorphthalsäureanhydrid auf wirtschaftliche Weise aus einer direkten Chlorierungsreaktion von Phthalsäureanhydrid zu gewinnen.

Darüber hinaus wird in der DE-C 638 200 die Trennung von chlorierten Phthalsäureanhydriden beschrieben, bei dem die o-substituierten von den o-unsubstituierten Chlorphthalsäureanhydriden dadurch getrennt werden, daß man nach der Behandlung mit H₂SO₄ die unveränderten Anhydride mit Toluol extrahiert.

Aufgabe der Erfindung war es demgemäß, ein Herstellungsverfahren für 3-Chlorphthalsäureanhydrid zu schaffen, das nicht auf einer vielstufigen Synthese beruht und auch im übrigen wirtschaftlich ist.

Diese Aufgabe wird gelöst durch ein Verfahren zur Herstellung von reinem 3-Chlorphthalsäureanhydrid aus einem Ausgangsgemisch, das neben 3-Chlorphthalsäureanhydrid 4,5-Di-chlorphthalsäureanhydrid enthält. Das erfindungsgemäße verfahren ist dann dadurch gekennzeichnet ist, daß aus dem Ausgangsgemisch zur Trennung von 3-Chlorphthalsäureanhydrid und 4,5-Dichlorphthalsäureanhydrid zunächst in einer Destillationsstufe (a) ein Gernisch von 3-Chlorphthalsäureanhydrid und 4,5-Dichlorphthalsäureanhydrid abdestilliert wird, so daß der erhaltene Sumpf frei von 4,5-Dichlorphthalsäureanhydrid ist, und anschließend in einer Destillationsstufe (b) 3-Chlorphthalsäureanhydrid durch Destillation aus dem erhaltenen Sumpf gewonnen wird.

Bevorzugte Ausführungsformen sind in den Unteransprüchen und der folgenden Beschreibung angegeben.

Unter den Begriffen reines 3-Chlorphthalsäureanhydrid bzw. frei von 4,5-Dichlorphthalsäureanhydrid ist - siehe Beispiele weiter unten - zu verstehen, daß diese Verbindungen "im wesentlichen rein" bzw. "im wesentlichen frei" sind, d.h. daß der Gehalt an 4,5-Dichlorphthalsäureanhydrid im Endprodukt noch 0,1 oder 0,2 Gew.-% betragen kann; entsprechende Werte sind auch für den Gehalt im Sumpf vor Einsatz der Destillationsstufe (b) noch möglich.

Überraschenderweise ist gefunden worden, daß entgegen der von A. Zweig und M. Epstein gewiesenen Richtung die destillative Trennung von 3-Chlorphthalsäureanhydrid und 4,5-Dichlorphthalsäureanhydrid möglich ist. Hierzu müssen geringe, die Wirtschaftlichkeit des Verfahrens kaum beeinflussende Verluste an 3-Chlorphthalsäureanhydrid in Kauf genommen werden. Diese Verluste sind umso niedriger, je niedriger der Gehalt des Chlorierungsgemischs an 4,5-Dichlorphthalsäureanhydrid ist, der, wie oben dargelegt, vom Umsatz des Phthalsäureanhydrids bei der Chlorierung abhängt.

Bevorzugterweise wird das Verfahren so durchgeführt, daß das Gemisch von 3-Chlorphthalsäureanhydrid und 4,5-Dichlorphthalsäureanhydrid bei einem Druck von 2,5 bis 80 kPa, bevorzugt von 3 bis 20 kPa, und einer Temperatur von 185 bis 300°C, bevorzugt 190 bis 250°C, in der ersten Destillationsstufe abdestilliert wird.

Aus dem Chlorierungsgemisch einer Chlorierung von Phthalsäureanhydrid mit Lewis-Säure-Katalysatoren können, wie bekannt und wie aufgrund der Dampfdrücke vorhersehbar, unumgesetztes Phthalsäureanhydrid und 4-Chlorphthalsäureanhydrid nacheinander abdestilliert oder gemeinsam abdestilliert werden, wenn weder die Rückführung von Phthalsäureanhydrid noch die Gewinnung von 4-Chlorphthalsäureanhydrid gewünscht wird. Diese Destillationen können kontinuierlich oder diskontinuierlich erfolgen, und die Reinheit der Komponenten kann durch den Destillationsaufwand bestimmt werden. Nach diesen Destillationsschritten verbleibt im Sumpf ein Produktgemisch, welches bei nicht zu hohen Umsatzraten von Phthalsäureanhydrid überwiegend aus 3-Chlorphthalsäureanhydrid und 4,5-Dichlorphthalsäureanhydrid sowie weiter aus 3,4- und 3,6-Dichlorphthalsäureanhydrid und geringen Mengen trichlorierter Produkte zusammengesetzt ist.

Die weitere Destillation dieses Gemisches liefert bei hinreichender Trennstufenzahl und hinreichendem Rücklauf überraschenderweise ein Kopfprodukt, in dem das Verhältnis von 4,5-Dichlorphthalsäureanhydrid zu 3-Chlorphthalsäureanhydrid gegenüber dem Verhältnis im Sumpf deutlich erhöht ist. Diese Fraktion kann erhalten werden, bis im Sumpf der Kolonne im wesentlichen kein 4,5-Dichlorphthalsäureanhydrid mehr vorhanden ist. In Anbetracht der identischen Siedepunkte und des gleichen Verlaufs der Dampfdrücke war dieses Verhalten des Gemisches nicht vorhersehbar und steht auch im Gegensatz zur Lehre von A. Zweig und M. Epstein, loc. cit.

Das 4,5-Dichlorphthalsäureanhydrid-freie Sumpfprodukt besteht aus 3-Chlorphthalsäureanhydrid, den übrigen dichlorierten Verbindungen und höher chlorierten Produkten. Die Reindarstellung von 3-Chlorphthalsäureanhydrid findet in einem anschließenden kontinuierlichen oder diskontinuierlichen Destillationsschritt statt, in dem es von der nächsten höher siedenden Komponente, 3,4-Dichlorphthalsäureanhydrid, quantitativ abgetrennt wird. 3,6-Dichlorphthalsäureanhydrid und trichlorierte Verbindungen weisen noch höhere Siedepunkte als 3,4-Dichlorphthalsäureanhydrid auf und sind somit für die Herstellung von reinem 3-Chlorphthalsäureanhydrid durch Destillation ohne Bedeutung. Das Sumpfprodukt des beschriebenen Destillationsverfahrens kann nach Gewinnung von 3-Chlorphthalsäureanhydrid gemäß einer weiteren Ausführungsform der Erfindung in Perchlorphthalsäureanhydrid überführt werden.

Die Größe der Zwischenfraktion aus 4,5-Dichlorphthalsäureanhydrid und 3-Chlorphthalsäureanhydrid wird durch den Gehalt an 4,5-Dichlorphthalsäureanhydrid im Chlorierungsproduktgemisch vorgegeben und kann ebenfalls zu dem als Farbstoffvorprodukt benutzten Perchlorphthalsäureanhydrid weiterverarbeitet werden.

Somit gestattet die Erfindung die Herstellung von reinem 3-Chlorphthalsäureanhydrid und, falls gewünscht, die gleichzeitige Herstellung von reinem 4-Chlorphthalsäureanhydrid in guter Ausbeute, insbesondere bezogen auf die Summe der beiden Isomeren, während die Bildung ggf. unerwünschter höher chlorierter Nebenprodukte durch einen nicht vollständigen Umsatz der Chlorierung von Phthalsäureanhydrid verhältnismäßig niedrig gehalten werden kann.

Im Laufe der Untersuchungen über das Produktgemisch einer Chlorierungsreaktion von Phthalsäureanhydrid wurden auch einige der in der Literatur angegebenen Siedepunkte an hochgereinigten Proben überprüft. Eine Gegenüberstellung erfolgt in der folgenden Tabelle III:

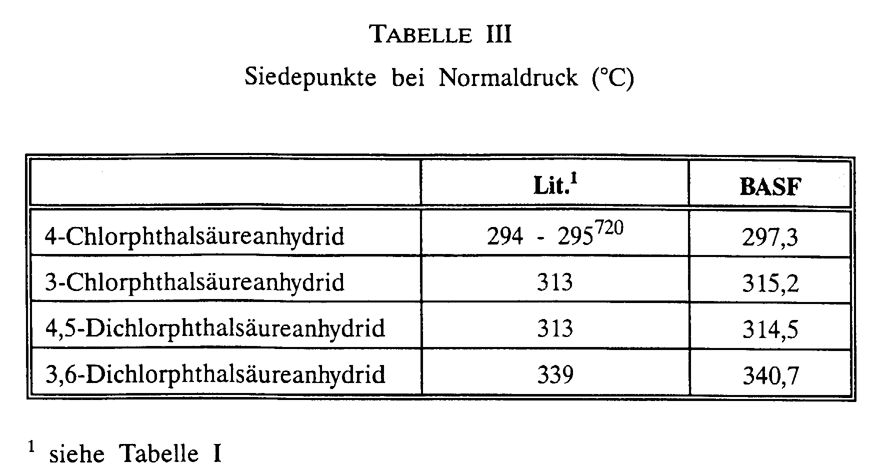

Die erfindungsgemäße Herstellung von 3-Chlorphthalsäureanhydrid in hoher Reinheit ist den anderen beschriebenen Verfahren, wie die Herstellung über die Nitroverbindung oder über die Oxidation von 3-Chlor-oxylol oder auch eine extraktive spätere Auftrennung wirtschaftlich weit überlegen.

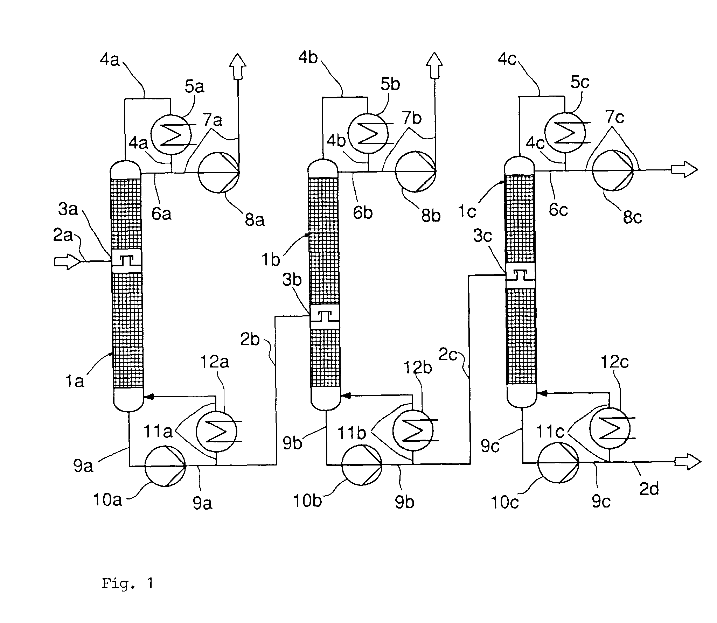

Eine schematische Darstellung einer für eine kontinuierliche Durchführung des erfindungsgemäßen Verfahrens geeigneten Apparatur ist der Zeichnung zu entnehmen. Dort sind drei Destillationskolonnen für die Durchführung des erfindungsgemäßen Verfahren mit 1a, 1b und 1c bezeichnet. Diese Kolonnen enthalten in bekannter Weise Füllkörper oder Rektifikationsböden, und zwar derart, daß die Kolonne 1a 25 Rektifikationsstufen, die Kolonne 1b 42 Rektifikationsstufen und die Kolonne 1c 54 Rektifikationsstufen aufweist. Die Zufuhrleitungen 2a, 2b und 2c treten in die Kolonnen 1a, 1b, 1c auf der 15., 8. bzw. 25. Rektifikations-stufe ein. Am Kopf der Kolonnen 1a, 1b, 1c führen die Leitungen 4a, 4b, 4c zu den Kondensatoren 5a, 5b, 5c und weiter zu den Leitungen 6a, 6b, 6c, die zu den Kolonnenköpfen zurückführen, sowie zu den Leitungen 7a, 7b, 7c, die über die Pumpen 8a, 8b, 8c zu Destillationsproduktbehältern (nicht dargestellt) führen. Die Leitungen 9a, 9b, 9c führen über die Pumpen 10a, 10b, 10c vom unteren Ende der Kolonnen 1a, 1b, 1c sowohl zu den Leitungen 11a, 11b, 11c, die über die Verdampfer 12a, 12b, 12c zu den unteren Kolonnenenden der Kolonnen 1a, 1b, 1c zurückführen, als auch zu den Zufuhrleitungen 2b, 2c, 2d der Kolonnen 1b, 1c und einer weiteren Kolonne (nicht dargestellt).

Die Durchführung des erfindungsgemäßen Verfahrens in dieser Anlage erfolgt in einem Ausführungsbeispiel der Erfindung in der Weise, daß zunächst ein lösungsmittelfreies Rohdestillat einer Chlorierungsreaktion von Phthalsäureanhydrid über die Zufuhrleitung 2a in die Kolonne la eingespeist wird. Am Kopf der Kolonne la wird bei 178,3°C und einem Druck von 0,058 bar über die Leitung 4a, den Kondensator 5a, die Leitung 7a unter Zuhilfenahme der Pumpe 8a ein Teil des Destillats, das im wesentlichen Phthalsäureanhydrid enthält, entnommen, während der größte Teil des Destillats nach Kondensation im Kondensator 5a über die Leitung 6a in die Kolonnen 1a zurückgeführt wird. Der Sumpf der Kolonne la wird, unter Zuhilfenahme der Pumpe 10a, durch Durchleiten durch die Leitungen 9a und 11a und durch den Verdampfer 12a bei 203,8°C gehalten; ein Teil des im wesentlichen Phthalsäureanhydridfreien Sumpfprodukts der Kolonne 1a wird über die Leitung 2b kontinuierlich der Kolonne 1b auf deren 8. Stufe zugeführt. Am Kopf der Kolonne 2b wird bei 176,3°C und einem Druck von 0,034 bar auf ähnliche Weise wie bei der Kolonne la 4-Chlorphthalsäureanhydrid abdestilliert, während der 4-Chlorphthalsäureanhydrid-freie Sumpf auf ähnliche Weise wie bei der Kolonne la bei 204,4°C gehalten sowie über die Leitung 2c der Kolonne lc auf deren 25. Stufe zugeführt wird. Am Kopf der Kolonne 1c wird bei 186,6°C und einem Druck von 0,03 bar ein Gemisch von 3-Chlorphthalsäureanhydrid und 4,5-Dichlorphthalsäureanhydrid entnommen. Der Sumpf der Kolonne 1c wird bei 205°C gehalten und als im wesentlichen 4,5-Dichlorphthalsäureanhydrid-freies Produkt, das das verbliebene 3-Chlorphthalsäureanhydrid enthält, über die Zufuhrleitung 2d einer weiteren Kolonne zugeführt, in der das verbliebene 3-Chlorphthalsäureanhydrid kontinuierlich oder diskontinuierlich auf übliche Weise praktisch quantitativ gewonnen werden kann.

Die folgenden Beispiele erläutern die Erfindung weiter. Die Chlorierungsreaktionen von Phthalsäureanhydrid wurden unter Einsatz von FeCl₃ als Katalysator ohne Verwendung eines Lösungsmittels in einer Schmelze bis zu dem in den Beispielen angegebenen Umsatz von Phthalsäureanhydrid durchgeführt. Bei diesen Reaktionen entstand bemerkenswerterweise jeweils etwas mehr 3-Chlorphthalsäureanhydrid als 4-Chlorphthalsäureanhydrid.

### Beispiel 1

Aus einem Rohdestillat einer Chlorierungsreaktion von Phthalsäureanhydrid, bestehend aus 57,00 Gew.-% Phthalsäureanhydrid, 18,30 Gew.-% 4-Chlorphthalsäureanhydrid, 20,50 Gew.-% 3-Chlorphthalsäureanhydrid, 0,90 Gew.-% 4,5-Dichlorphthalsäureanhydrid, 1,70 Gew.-% 3,6-Dichlorphthalsäureanhydrid und 1,60 Gew.-% höher siedenden Produkten, soll 3-Chlorphthalsäureanhydrid mit einem Restgehalt an 4,5-Dichlorphthalsäureanhydrid von 0,1 Gew.-% gewonnen werden. Das Rohdestillat wird in die Kolonne la auf der Höhe der 15. Stufe mit einer Beschickungsrate von 2000 kg/h eingespeist. Das Destillat, das über die Leitung 7a der Kolonne la entnommen wird, enthält 99,50 Gew.-% Phthalsäureanhydrid und 0,50 Gew.-% 4-Chlorphthalsäureanhydrid bei einem Rücklaufverhältnis von 24 [Rücklauf/Kopfabzug]. Aus der Kolonne 1b wird über die Leitung 7b bei einem Rücklaufverhältnis von 466 ein Destillat entnommen, das aus 0,50 Gew.-% Phthalsäureanhydrid und 99,50 Gew.-% 4-Chlorphthalsäureanhydrid sowie 10 ppm 3-Chlorphthalsäureanhydrid besteht. Der Sumpf der Kolonne 1b, der über die Zufuhrleitung 2c mit einer Beschickungsrate von 494,00 kg/h in die Kolonne 1c eingespeist wird, besteht aus 30 ppm 4-Chlorphthalsäureanhydrid, 83 Gew.-% 3-Chlorphthalsäureanhydrid, 3,60 Gew.-% 4,5-Dichlorphthalsäureanhydrid, 6,90 Gew.-% 3,6-Dichlorphthalsäureanhydrid sowie 6,50 Gew.-% höher siedenden Produkten. Bei einem Rücklaufverhältnis von 150 werden der Kolonne lc stündlich 29,80 kg eines Gemisches entnommen, das aus 496 ppm 4-Chlorphthalsäureanhydrid, 41,00 Gew.-% 3-Chlorphthalsäureanhydrid und 59,00 Gew.-% 4,5-Dichlorphthalsäureanhydrid besteht. Im Sumpf der Kolonne 1c befinden sich noch 0,10 Gew.-% 4,5-Dichlorphthalsäureanhydrid, 85,70 Gew.-% 3-Chlorphthalsäureanhydrid, 7,30 Gew.-% 3,6-Dichlorphthalsäureanhydrid und 6,90 Gew.-% höher siedende Produkte. Die Gewinnung des 3-Chlorphthalsäureanhydrids, das nur noch 0,1 Gew.-% 4,5-Dichlorphthalsäureanhydrid enthält, erfolgt daraus in der nächsten Kolonne praktisch quantitativ.

Dieses Beispiel belegt, daß auch bei wirtschaftlich interessanten Chlorierungsraten von Phthalsäureanhydrid sehr reines 3-Chlorphthalsäureanhydrid erhalten werden kann.

### Beispiel 2

Ein Rohdestillat wie in Beispiel 1 wurde auf gleiche Weise aufgearbeitet, außer daß ein Restgehalt von 0,2 Gew.-% an 4,5-Dichlorphthalsäureanhydrid in 3-Chlorphthalsäureanhydrid erhalten werden sollte und aus diesem Grund ein Rücklaufverhältnis am Kolonnenkopf der Kolonne 1c von 115 gewählt wurde. Das in diesem Beispiel dem Kopf der Kolonne 1c zu 29,20 kg/h entnommene Gemisch bestand aus 508 ppm 4-Chlorphthalsäureanhydrid, 41 Gew.-% 3-Chlorphthalsäureanhydrid und 59 Gew.-% 4,5-Dichlorphthalsäureanhydrid. Der Sumpf der Kolonne 1c bestand aus 85,60 Gew.-% 3-Chlorphthalsäureanhydrid, 0,20 Gew.-% 4,5-Dichlorphthalsäureanhydrid, 7,30 Gew.-% 3,6-Dichlorphthalsäureanhydrid und 6,90 Gew.-% höher siedenden Produkten. 3-Chlorphthalsäureanhydrid wird dann mit dem im Sumpf vorgegebenen Restgehalt an 4,5-Dichlorphthalsäureanhydrid praktisch quantitativ aus demselben gewonnen.

Dieses Beispiel belegt, daß die Reinheit des erhaltenen 3-Chlorphthalsäureanhydrids durch das Rücklaufverhältnis am Kopf der Kolonne 1c gesteuert werden kann.

### Beispiel 3

Aus einem Rohdestillat einer Chlorierungsreaktion von Phthalsäureanhydrid, bestehend aus 78,50 Gew.-% Phthalsäureanhydrid, 9,20 Gew.-% 4-Chlorphthalsäureanhydrid, 11,40 Gew.-% 3-Chlorphthalsäureanhydrid, 0,28 Gew.-% 4,5-Dichlorphthalsäureanhydrid, 0,41 Gew.-% 3,6-Dichlorphthalsäureanhydrid und 0,21 Gew.-% höher siedenden Produkten, soll 3-Chlorphthalsäureanhydrid mit einem Restgehalt an 4,5-Dichlorphthalsäureanhydrid von 0,1 Gew.-% gewonnen werden. Das Rohdestillat wird in die Kolonne 1a auf der Höhe der 15. Stufe mit einer Beschickungsrate von 2000 kg/h eingespeist. Das Destillat, das über die Leitung 7a der Kolonne 1a entnommen wird, enthält 99,50 Gew.-% Phthalsäureanhydrid und 0,50 Gew.-% 4-Chlorphthalsäureanhydrid bei einem Rücklaufverhältnis von 17. Aus der Kolonne 1b wird über die Leitung 7b bei einem Rücklaufverhältnis von 312 ein Destillat entnommen, das aus 0,50 Gew.-% Phthalsäureanhydrid und 99,50 Gew.-% 4-Chlorphthalsäureanhydrid sowie 10 ppm 3-Chlorphthalsäureanhydrid besteht. Der Sumpf der Kolonne 1b, der über die Zufuhrleitung 2c mit einer Beschickungsrate von 246,00 kg/h in die Kolonne 1c eingespeist wird, besteht aus 30 ppm 4-Chlorphthalsäureanhydrid, 92,70 Gew.-% 3-Chlorphthalsäureanhydrid, 2,30 Gew.-% 4,5-Dichlorphthalsäureanhydrid, 3,30 Gew.-% 3,6-Dichlorphthalsäureanhydrid sowie 1,70 Gew.-% höher siedenden Produkten. Bei einem Rücklaufverhältnis von 247 werden der Kolonne 1c stündlich 9,20 kg eines Gemisches entnommen, das aus 805 ppm 4-Chlorphthalsäureanhydrid, 41,30 Gew.-% 3-Chlorphthalsäureanhydrid und 58,70 Gew.-% 4,5-Dichlorphthalsäureanhydrid besteht. Im Sumpf der Kolonne lc befinden sich noch 0,10 Gew.-% 4,5-Dichlorphthalsäureanhydrid, 94,70 Gew.-% 3-Chlorphthalsäureanhydrid, 3,40 Gew.-% 3,6-Dichlorphthalsäureanhydrid und 1,80 Gew.-% höher siedende Produkte. Die Gewinnung des 3-Chlorphthalsäureanhydrids, das nur noch 0,1 Gew.-% 4,5-Dichlorphthalsäureanhydrid enthält, erfolgt daraus praktisch quantitativ in einer weiteren Kolonne.

Dieses Beispiel belegt, daß die Zwischenfraktion, die sowohl 3-Chlorphthalsäureanhydrid als 4,5-Dichlorphthalsäureanhydrid enthält, klein gehalten werden kann, wenn das Rohdestillat nur geringe Mengen an 4,5-Dichlorphthalsäureanhydrid enthält, und daß in diesem Fall außerdem zum Erhalt der gleichen Reinheit von 3-Chlorphthalsäureanhydrid ein geringeres Rücklaufverhältnis ausreichend ist.

### Beispiel 4

Ein Rohdestillat wie in Beispiel 3 wurde auf gleiche Weise wie in diesem Beispiel aufgearbeitet, außer daß ein Restgehalt von 0,2 Gew.-% an 4,5-Dichlorphthalsäureanhydrid in 3-Chlorphthalsäureanhydrid erhalten werden sollte und aus diesem Grund ein Rücklaufverhältnis am Kolonnenkopf der Kolonne 1c von 150 gewählt wurde. Das in diesem Beispiel dem Kopf der Kolonne 1c zu 8,80 kg/h entnommene Gemisch bestand aus 837 ppm 4-Chlorphthalsäureanhydrid, 40,91 Gew.-% 3-Chlorphthalsäureanhydrid und 59,09 Gew.-% 4,5-Dichlorphthalsäureanhydrid. Der Sumpf bestand aus 94,60 Gew.-% 3-Chlorphthalsäureanhydrid, 0,20 Gew.-% 4,5-Dichlorphthalsäureanhydrid, 3,40 Gew.-% 3,6-Dichlorphthalsäureanhydrid und 1,80 Gew.-% höher siedenden Produkten. 3-Chlorphthalsäureanhydrid wurde daraus mit einem Restgehalt von 0,20 Gew.-% 4,5-Dichlorphthalsäureanhydrid praktisch quantitativ gewonnen.

Dieses Beispiel belegt wiederum, daß die Reinheit des erhaltenen 3-Chlorphthalsäureanhydrids allein durch das Rücklaufverhältnis am Kopf der Kolonne lc gesteuert werden kann.

## Patentansprüche

1. Verfahren zur Herstellung von reinem 3-Chlorphthalsäureanhydrid aus einem Ausgangsgemisch, das neben 3-Chlorphthalsäureanhydrid 4,5-Dichlorphthalsäureanhydrid enthält, **dadurch gekennzeichnet**, daß aus dem Ausgangsgemisch zur Trennung von 3-Chlorphthalsäureanhydrid und 4,5-Dichlorphthalsäureanhydrid zunächst in einer Destillationsstufe (a) ein Gemisch von 3-Chlorphthalsäureanhydrid und 4,5-Dichlorphthalsäureanhydrid abdestilliert wird, so daß der erhaltene Sumpf frei von 4,5-Dichlorphthalsäureanhydrid ist, und anschließend in einer Destillationsstufe (b) 3-Chlorphthalsäureanhydrid durch Destillation aus dem erhaltenen Sumpf gewonnen wird.

2. Verfahren nach Anspruch 1, dadurch gekennzeichnet, daß die Reinheit des 3-Chlorphthalsäureanhydrids durch das Rücklaufverhältnis bei der Abtrennung des Gemisches von 3-Chlorphthalsäureanhydrid und 4,5-Dichlorphthalsäureanhydrid in der Destillationsstufe (a) gesteuert wird.

3. Verfahren nach einem der Ansprüche 1 oder 2, dadurch gekennzeichnet, daß das Gemisch von 3-Chlorphthalsäureanhydrid und 4,5-Dichlorphthalsäureanhydrid bei einem Druck von 2,5 bis 80 kPa, bevorzugt von 3 bis 20 kPa, und einer Temperatur von 185 bis 300°C, bevorzugt 190 bis 250°C, in der ersten Destillationsstufe abdestilliert wird.

4. Verfahren nach einem der Ansprüche 1 bis 3, dadurch gekennzeichnet, daß das Ausgangsgemisch durch unvollständige Chlorierung von Phthalsäureanhydrid hergestellt wird, woraus unumgesetztes Phthalsäureanhydrid durch kontinuierliche oder diskontinuierliche Destillation abgetrennt worden ist.

5. Verfahren nach Anspruch 4, dadurch gekennzeichnet, daß das Ausgangsgemisch unter Verwendung von FeCl₃ als Katalysator ohne Lösungsmittel in einer Schmelze hergestellt wird.

6. Verfahren nach einem der Ansprüche 4 oder 5, dadurch gekennzeichnet, daß das Phthalsäureanhydrid in die Chlorierungsstufe zur Herstellung des Ausgangsgemischs zurückgeführt wird.

7. Verfahren nach einem der Ansprüche 1 bis 6, dadurch gekennzeichnet, daß das in der Destillationsstufe (a) gewonnene Gemisch von 3-Chlorphthalsäureanhydrid und 4,5-Chlorphthalsäureanhydrid zu Perchlorphthalsäureanhydrid weiterverarbeitet wird.

8. Verfahren nach einem der Ansprüche 1 bis 7, dadurch gekennzeichnet, daß die nach der Destillationsstufe (b) im Sumpf verbleibenden Chlorierungsprodukte zu Perchlorphthalsäureanhydrid weiterverarbeitet werden.

9. Verfahren nach einem der vorhergehenden Ansprüche, dadurch gekennzeichnet, daß Phthalsäureanhydrid mit partiellem Umsatz chloriert wird, unumgesetztes Phthalsäureanhydrid durch kontinuierliche oder diskontinuierliche Destillation als Kopfprodukt abdestilliert wird, danach das erzeugte 4-Chlorphthalsäureanhydrid kontinuierlich oder diskontinuierlich als zweites Kopfprodukt abdestilliert wird, danach eine Mischung aus 4,5-Dichlorphthalsäureanhydrid und 3-Chlorphthalsäureanhydrid kontinuierlich oder diskontinuierlich als drittes Kopfprodukt abdestilliert wird, sowie dann reines 3-Chlorphthalsäureanhydrid kontinuierlich oder diskontinuierlich durch Destillation gewonnen wird, wobei vorzugsweise unumgesetztes Phthalsäureanhydrid und 4-Chlorphthalsäureanhydrid gemeinsam kontinuierlich oder diskontinuierlich abdestilliert werden, bevor die Gewinnung von 3-Chlorphthalsäureanhydrid durchgeführt wird, und wobei besonders bevorzugterweise unumgesetztes Phthalsäureanhydrid in die Chlorierung zurückgeführt wird.

## Claims

1. A process for preparing pure 3-chlorophthalic anhydride from a starting mixture which contains 4,5-dichlorophthalic anhydride in addition to 3-chlorophthalic anhydride, which comprises first distilling off a mixture of 3-chlorophthalic anhydride and 4,5-dichlorophthalic anhydride from the starting mixture for the separation of 3-chlorophthalic anhydride and 4,5-dichlorophthalic anhydride in a distillation step (a), so that the bottom obtained is free of 4,5-dichlorophthalic anhydride, and then, by distillation of the bottom obtained, recovering 3-chlorophthalic anhydride in a distillation step (b).

2. A process as claimed in claim 1, wherein the purity of the 3-chlorophthalic anhydride is controlled by the reflux ratio during the separation of the mixture of 3-chlorophthalic anhydride and 4,5-dichlorophthalic anhydride in the distillation step (a).

3. A process as claimed in one of claims 1 or 2, wherein the mixture of 3-chlorophthalic anhydride and 4,5-dichlorophthalic anhydride is distilled off in the first distillation step at from 2.5 to 80 kPa, preferably 3 to 20 kPa, and from 185 to 300°C, preferably 190 to 250°C.

4. A process as claimed in one of claims 1 to 3, wherein the starting mixture is prepared by incomplete chlorination of phthalic anhydride, from which unreacted phthalic anhydride has been removed by continuous or batchwise distillation.

5. A process as claimed in claim 4, wherein the starting mixture is prepared without solvent in a melt using FeCl₃ as a catalyst.

6. A process as claimed in one of claims 4 or 5, wherein the phthalic anhydride is fed back into the chlorination step for preparing the starting mixture.

7. A process as claimed in one of claims 1 to 6, wherein the mixture of 3-chlorophthalic anhydride and 4,5-dichlorophthalic anhydride obtained in the distillation step (a) is processed further to give perchlorophthalic anhydride.

8. A process as claimed in one of claims 1 to 7, wherein the chlorination products remaining in the bottom after the distillation step (b) are processed further to give perchlorophthalic anhydride.

9. A process as claimed in one of the preceding claims, wherein phthalic anhydride is chlorinated with partial conversion, unreacted phthalic anhydride is distilled off as a top product by continuous or batchwise distillation, then the 4-chlorophthalic anhydride produced is distilled off continuously or batchwise as a second top product, then a mixture of 4,5-dichlorophthalic anhydride and 3-chlorophthalic anhydride is distilled off continuously or batchwise as a third top product, and then pure 3-chlorophthalic anhydride is recovered continuously or batchwise by distillation, unreacted phthalic anhydride and 4-chlorophthalic anhydride preferably being distilled off together continuously or batchwise before the recovery of 3-chlorophthalic anhydride is carried out, and unreacted phthalic anhydride particularly preferably being fed back into the chlorination.

## Revendications

1. Procédé de préparation d'anhydride d'acide 3-chlorophtalique pur à partir d'un mélange de départ contenant de l'anhydride d'acide 4,5-dichlorophtalique en plus de l'anhydride d'acide 3-chlorophtalique, caractérisé en ce que, afin de séparer l'anhydride d'acide 3-chlorophtalique de l'anhydride d'acide 4,5-dichlorophtalique, on élimine tout d'abord par distillation du mélange de départ, un mélange d'acide 3-chlorophtalique et d'anhydride d'acide 4,5-dichlorophtalique dans une première étape de distillation (a), de telle sorte que le produit de fond de colonne obtenu soit exempt d'anhydride d'acide 4,5-dichlorophtalique, puis on récupère l'anhydride d'acide 3-chlorophtalique par distillation du produit de fond de colonne obtenu, dans une deuxième étape de distillation (b).

2. Procédé selon la revendication 1, caractérisé en ce que l'on régule la pureté de l'anhydride d'acide 3-chlorophtalique au moyen du rapport de retour lors de la séparation du mélange d'anhydride d'acide 3-chlorophtalique et d'anhydride d'acide 4,5-dichlorophtalique dans la première étape de distillation (a).

3. Procédé selon l'une ou l'autre des revendications 1 ou 2, caractérisé en ce que, lors de la première étape de distillation, on élimine par distillation le mélange d'anhydride d'acide 3-chlorophtalique et d'anhydride d'acide 4,5-dichlorophtalique sous une pression de 2,5-80 kPa, de préférence 3-20 kPa, et à une température de 185-300°C, de préférence 190-250°C.

4. Procédé selon l'une quelconque des revendications 1 à 3, caractérisé en ce que le mélange de départ est produit par la chloration incomplète d'anhydride phtalique, au cours de laquelle l'anhydride phtalique n'ayant pas réagi a été éliminé par distillation continue ou discontinue.

5. Procédé selon la revendication 4, caractérisé en ce que le mélange de départ est produit dans la masse, sans solvant, avec utilisation de FeCl₃ en tant que catalyseur.

6. Procédé selon l'une ou l'autre des revendications 4 ou 5, caractérisé en ce que l'anhydride phtalique est renvoyé dans l'étape de chloration pour la préparation du mélange de départ.

7. Procédé selon l'une quelconque des revendications 1 à 6, caractérisé en ce que le mélange d'anhydride d'acide 3-chlorophtalique et d'anhydride d'acide 4,5-dichlorophtalique récupéré lors de l'étape de distillation (a) est ensuite transformé en anhydride d'acide perchlorophtalique.

8. Procédé selon l'une quelconque des revendications 1 à 7, caractérisé en ce que les produits de chloration restant en fond de colonne après l'étape de distillation (b) sont ensuite transformés en anhydride d'acide perchlorophtalique

9. Procédé selon l'une quelconque des revendications précédentes, caractérisé en ce que l'on chlore de l'anhydride d'acide phtalique avec une conversion partielle, on élimine par distillation continue ou discontinue l'anhydride d'acide phtalique n'ayant pas réagi, en tant que produit obtenu en tête de colonne, puis on élimine par distillation continue ou discontinue l'anhydride d'acide 4-chlorophtalique, en tant que deuxième produit obtenu en tête de colonne, puis on élimine par distillation continue ou discontinue un mélange d'anhydride d'acide 3-chlorophtalique et d'anhydride d'acide 4,5-dichlorophtalique, en tant que troisième produit obtenu en tête de colonne, puis on récupère de l'anhydride d'acide 3-chlorophtalique pur par distillation continue ou discontinue, dans lequel on élimine, de préférence, l'anhydride d'acide phtalique n'ayant pas réagi et l'anhydride d'acide 4-chlorophtalique ensemble par distillation continue ou discontinue avant que la récupération de l'anhydride d'acide 3-chlorophtalique ne soit effectuée, et dans lequel, de façon plus particulièrement préférée, on renvoie l'anhydride d'acide phtalique n'ayant pas réagi dans la chloration.
